# EUROPEAN PATENT APPLICATION

(11) **EP 1 803 701 A1**
(43) Date of publication of application: **04.07.2007**
(21) Application number: 06026482.7
(22) Date of filing: 20.12.2006
(51) Int. Cl.: C07C 41/32, C07C 43/162, C07D 493/04

(54) **Process for the preparation of vinyl ether compounds**

(30) Priority: 27.12.2005 JP 2005376000
(71) Applicant: DAICEL CHEMICAL INDUSTRIES, LTD., Sakai-shi, Osaka 590-8501 (JP)
(72) Inventor: Iwahama, Takahiro, c/o Research Center, Aboshi-ku, Himeji-shi, Hyogo 671-1283 (JP); Ishii, Yasutaka, Tatsuki-shi, Osaka 569-1112 (JP)
(74) Representative: Grünecker, Kinkeldey, Stockmair & Schwanhäusser Anwaltssozietät

(57) **Abstract**

A vinyl ether compound represented by following Formula (2) : 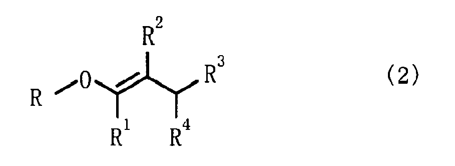 wherein R represents an organic group; and R¹, R², R³ and R⁴ are the same as or different from one another and each represent a hydrogen atom or an organic group, is produced by isomerizing an allyl ether compound in the presence of a base and a compound of a Group VIII element of the Periodic Table of Elements, which allyl ether compound is represented by following Formula (1): 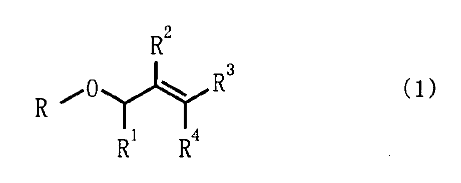 wherein R, R¹, R², R³ and R⁴ are as defined above.

## Description

### BACKGROUND OF THE INVENTION

### 1. Field of the Invention

The present invention relates to processes for the preparation of vinyl ether compounds. Such vinyl ether compounds are useful as raw materials typically for pharmaceutical drugs and agricultural chemicals, and raw materials for polymers, such as cationically polymerizable materials.

### 2. Description of the Related Art

Vinyl ether compounds are useful as raw materials for fine chemicals such as pharmaceutical drugs and agricultural chemicals, and as raw materials for polymers such as resist resins, optical resins, transparent resins, and crosslinking resins. Among them, vinyl ether compounds having nonaromatic cyclic skeletons such as alicyclic skeletons and lactone skeletons are promising material monomers for constituting resist resins, because when they are used as comonomers for polymers, the resulting polymers may have improved transparency and improved resistance to dry etching. Vinyl ether compounds each having plural vinyl groups are suitable as material monomers for crosslinking resins, because they can impart excellent solvent resistance to the resulting resins. In addition, vinyl ether compounds develop less odor and less irritant to the skin than acrylic compounds and can be more satisfactorily handled and worked. Commercially available vinyl ether compounds, however, are more expensive and more limited in their types than acrylic compounds as monomers, and may not respond to present needs sufficiently.

Processes for the preparation of vinyl ether compounds include a process of reacting acetylene and an alcohol in the presence of a catalytic alkali metal hydroxide or alkali metal alcoholate. This process is disadvantageous, because it uses, as a raw material, acetylene which is difficult to handle. Another process for the preparation of vinyl ether compound is a process of reacting an alcohol and a vinyl ester at temperatures of -75°C to -15°C in the presence of a mercury salt of strong acid (U.S. Pat. No. 2,579,411). This process, however, is not preferred as an industrial production process, because it uses very toxic mercury and carries out the reaction at very low temperatures.

Yet another process for the preparation of vinyl ether compound is a process of reacting a vinyl ester of carboxylic acid and a hydroxy compound in the presence of a transition element compound such as an iridium compound (Japanese Unexamined Patent Application Publication (JP-A) No. 2003-73321). According to this process, there may be easily and conveniently yielded a target vinyl ether compound under mild conditions. The process, however, is still insufficient in yield.

### SUMMARY OF THE INVENTION

Under these circumstances, it is desirable to provide a process for efficiently preparing vinyl ether compounds using an isomerization reaction under mild conditions.

After intensive investigations, the present inventors have found that an isomerization reaction of an allyl ether compound may be enhanced under mild conditions by carrying out the reaction in the presence of a base using a specific metal element compound as a catalyst.

According to an embodiment of the present invention, there is provided a process for producing a vinyl ether compound represented by following Formula (2): 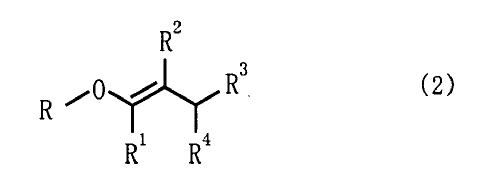 wherein R represents an organic group; and R¹, R², R³ and R⁴ are the same as or different from one another and each represent a hydrogen atom or an organic group, the process including the step of isomerizing an allyl ether compound in the presence of a base and a compound of a Group VIII element of the Periodic Table of Elements, the allyl ether compound represented by following Formula (1): 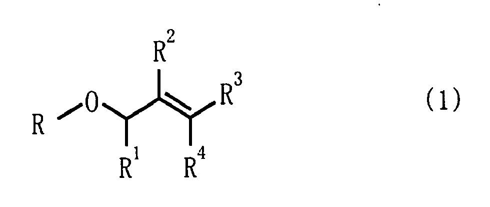 wherein R, R¹, R², R³ and R⁴ are as defined above. The compound of a Group VIII element of the Periodic Table of Elements may include an iridium compound.

A process according to an embodiment of the present invention uses a base and a compound of a Group VIII element of the Periodic Table of Elements as catalysts, may allow an isomerization reaction of an allyl ether compound to proceed in a short time; and may thereby efficiently yield a vinyl ether compound. A process according to an embodiment of the present invention can be satisfactorily used in a wide variety of applications and is useful in the preparation of vinyl ethers. Such vinyl ethers are useful as raw materials for fine chemicals such as pharmaceutical drugs and agricultural chemicals and as raw materials for polymers such as resist resins, optical resins, transparent resins, and crosslinking resins.

### DESCRIPTION OF THE PREFERRED EMBODIMENTS

### [Compound of Group VIII element of the Periodic Table of Element]

A compound of a Group VIII element of the Periodic Table of Elements (including an elementary substance of the element) is used as a catalyst according to an embodiment of the present invention. Each of such compounds of Group VIII elements of the Periodic Table of Elements (hereinafter also briefly referred to as "Group VIII element compound(s)") can be used alone or in combination. Group VIII elements include, for example, iron, cobalt, nickel, ruthenium, rhodium, palladium, osmium, iridium, and platinum. Among them, platinum group elements including ruthenium, rhodium, palladium, osmium, iridium, and platinum are preferred, of which iridium is typically preferred for its excellent catalytic activity and low cost.

Examples of compounds of a Group VIII element include inorganic compounds of the element, such as elementary substance (metal), oxides, sulfides, hydroxides, halides such as fluorides, chlorides, bromides, and iodides, sulfates, oxoacids or salts thereof, and inorganic complexes each containing the Group VIII element; and organic compounds of the element, such as cyanides, salts of organic acids, such as acetates, and organic complexes. Of these, organic complexes are preferred. Ligands of complexes include known ligands. A Group VIII element in a Group VIII element compound may have a valency of about 0 to about 6, and preferably about 0 to about 3. An iridium compound typically preferably has a valency of iridium of 1 or 3.

Taking iridium as an example, representative examples of Group VIII element compounds include inorganic compounds including metal iridium, iridium oxides, iridium sulfides, iridium hydroxides, iridium fluorides, iridium chlorides, iridium bromides, iridium iodides, iridium sulfates, iridium acids or salts thereof such as potassium iridate, inorganic iridium complexes such as hexaammineiridium(III) salts and chloropentaammineiridium(III) salts; and organic compounds including iridium cyanides, organic iridium complexes such as tris(acetylacetonato)iridium, dodecacarbonyltetrairidium(0), chlorotricarbonyliridium(I), di-µ-chlorotetrakis(cyclooctene)diiridium(I), di-µ-chlorotetrakis(ethylene)diiridium(I), di-µ-chlorobis(1,5-cyclooctadiene)diiridium(I), di-µ-chlorodichlorobis(pentamethylcyclopentadienyl)diiridium(III), trichlorotris(triethylphosphine)iridium(III), pentahydridobis(trimethylphosphine)iridium(V), chlorocarbonylbis(triphenylphosphine)iridium(I), chloroethylenebis(triphenylphosphine)iridium(I), (pentamethylcyclopentadienyl)dicarbonyliridium(I), bis[1,2-bis(diphenylphosphino)ethane]iridium(I) chloride, pentamethylcyclopentadienylbis(ethylene)iridium(I), carbonylmethylbis(triphenylphosphine)iridium(I), (1,5-cyclooctadiene)(diphosphine)iridium(I) halides, 1,5-cyclooctadiene(1,2-bis(diphenylphosphino)ethane)iridium(I) hexafluorophosphate, (1,5-cyclooctadiene)bis(trialkylphosphine)iridium(I) halides, bis(1,5-cyclooctadiene)iridium tetrafluoroborate, and (1,5-cyclooctadiene)(acetonitrile)iridium tetrafluoroborate.

Preferred iridium compounds include iridium complexes. Among them, organic iridium complexes are more preferred, of which typically preferred are organic iridium complexes having, as ligands, unsaturated hydrocarbons, nitriles, and/or ethers. Examples of unsaturated hydrocarbons include cyclopentene, dicyclopentadiene, cyclooctene, 1,5-cyclooctadiene, ethylene, pentamethylcyclopentadiene, benzene, and toluene. The nitriles include acetonitrile, and the ethers include tetrahydrofuran. Examples of preferred organic iridium complexes include di-µ-chlorotetrakis(cyclooctene)diiridium(I), di-µ-chlorotetrakis(ethylene)diiridium(I), di-µ-chlorobis(1,5-cyclooctadiene)diiridium(I), bis(1,5-cyclooctadiene)iridium tetrafluoroborate, and (1,5-cyclooctadiene)(acetonitrile)iridium tetrafluoroborate. Cationic iridium complexes, such as bis(1,5-cyclooctadiene)iridium tetrafluoroborate and (1,5-cyclooctadiene) (acetonitrile)iridium tetrafluoroborate, are preferably used according to an embodiment of the present invention. Each of iridium compounds can be used alone or in combination. It is also acceptable to use one or more iridium compounds in combination with one or more compounds of other Group VIII elements of the Periodic Table of Elements.

Examples of other Group VIII element compounds than iridium compounds include compounds corresponding to the iridium compounds, such as dichloro(1,5-cyclooctadiene)ruthenium, dichloro (1,5-cyclooctadiene)platinum, and dichlorobis (1,5-cyclooctadiene)dirhodium. Of other Group VIII element compounds than iridium compounds, preferred are organic complexes having, as ligands, unsaturated hydrocarbons such as cyclopentene, dicyclopentadiene, cyclooctene, 1,5-cyclooctadiene, ethylene, pentamethylcyclopentadiene, benzene, and toluene; nitriles such as acetonitrile; and ethers such as tetrahydrofuran. Among them, cationic complexes are often used.

A Group VIII element compound can be used as intact or as being supported by a carrier. Examples of the carrier include carriers generally used for supporting catalysts, including inorganic metal oxides such as silica, alumina, silica-alumina, zeolite, titania, and magnesia; and activated carbon. The amount of a Group VIII element compound in a carrier-supported catalyst is, for example, about 0.1 to about 50 percent by weight, preferably about 1 to about 20 percent by weight, to the weight of the carrier. Such a catalyst can be supported by a carrier according to a procedure in related art, such as impregnation, precipitation, or ion exchange.

The amount of a Group VIII element compound is, for example, about 0.0001 to about 1 mole, preferably about 0.001 to about 0.3 mole, and more preferably about 0.005 to about 0.1 mole, to 1 mole of a compound represented by Formula (1) used as a reaction component.

### [Base]

A reaction is carried out in the presence of a base according to an embodiment of the present invention. By satisfying this, an isomerization reaction is more speeded up to thereby efficiently yield a vinyl ether in a shorter time than a reaction using a catalytic Group VIII element compound alone. Bases herein include inorganic bases including hydroxides such as sodium hydroxide and potassium hydroxide, and carbonates such as sodium carbonate and cesium carbonate; and organic bases such as sodium acetate and sodium ethoxide. Among them, inorganic bases are preferred, of which carbonates such as sodium carbonate and cesium carbonate are more preferred. The amount of a base is, for example, about 0.001 to about 3 moles, and preferably about 0.005 to about 2 moles, to 1 mole of an allyl ether compound represented by Formula (1).

### [Allyl ether compound]

Organic groups as R, R¹, R², R³, and R⁴ in Formula (1) can be any organic groups that may not adversely affect the reaction, such as organic groups which are inert under reaction conditions in a process according to an embodiment of the present invention. Examples of organic groups include halogen atoms; hydrocarbon groups; heterocyclic groups; substituted oxycarbonyl groups such as alkoxycarbonyl groups, aryloxycarbonyl groups, aralkyloxycarbonyl groups, and cycloalkyloxycarbonyl groups; carboxyl group; substituted or unsubstituted carbamoyl groups; cyano group; nitro group; sulfur acid groups; sulfur acid ester groups; acyl groups including aliphatic acyl groups such as acetyl group, and aromatic acyl groups such as benzoyl group; alkoxy groups including alkoxy groups each having one to six carbon atoms, such as methoxy group and ethoxy group; N,N-disubstituted amino groups such as N,N-dimethylamino group and piperidino group; and groups each containing two or more of these groups combined with each other. The carboxyl group and other groups may be protected by protecting groups used in organic syntheses in related art. The halogen atoms include fluorine, chlorine, bromine and iodine atoms. Of these organic groups, preferred are hydrocarbon groups and heterocyclic groups.

The hydrocarbon groups and heterocyclic groups also include substituted hydrocarbon groups and substituted heterocyclic groups. The hydrocarbon groups include aliphatic hydrocarbon groups, alicyclic hydrocarbon groups, aromatic hydrocarbon groups, and groups each containing two or more of these groups. Examples of aliphatic hydrocarbon groups include alkyl groups having about one to about twenty carbon atoms, such as methyl, ethyl, propyl, isopropyl, butyl, isobutyl, s-butyl, t-butyl, pentyl, hexyl, decyl, and dodecyl groups, of which alkyl groups having about one to about ten carbon atoms are preferred, and those having about one to about three carbon atoms are more preferred; alkenyl groups having about two to about twenty carbon atoms, such as vinyl, allyl, and 1-butenyl groups, of which those having about two to about ten carbon atoms are preferred, and those having about two or three carbon atoms are more preferred; and alkynyl groups having about two to about twenty carbon atoms, such as ethynyl and propynyl groups, of which those having about two to about ten carbon atoms are preferred, and those having about two or three carbon atoms are more preferred.

Alicyclic hydrocarbon groups include, for example, cycloalkyl groups having about three to about twenty members, such as cyclopropyl, cyclobutyl, cyclopentyl, cyclohexyl, and cyclooctyl groups, of which those having about three to about fifteen members are preferred, and those having about five to about eight members are more preferred; cycloalkenyl groups having about three to about twenty members, such as cyclopentenyl and cyclohexenyl groups, of which those having about three to about fifteen members are preferred, and those having about five to about eight members are more preferred; and bridged hydrocarbon groups such as perhydronaphth-1-yl group, norbornyl group, adamantyl group, and tetracyclo[4.4.0.1^{2,5}.1^{7,10}]dodec-3-yl group. Aromatic hydrocarbon groups herein include aromatic hydrocarbon groups having about six to about fourteen carbon atoms, such as phenyl and naphthyl groups, of which those having about six to about ten carbon atoms are preferred.

Hydrocarbon groups each containing an aliphatic hydrocarbon group and an alicyclic hydrocarbon group combined with each other include cycloalkyl-alkyl groups including cycloalkyl-alkyl groups whose cycloalkyl moiety has about three to about twenty carbon atoms and whose alkyl moiety has about one to about four carbon atoms, such as cyclopentylmethyl, cyclohexylmethyl, and 2-cyclohexylethyl groups. Hydrocarbon groups each containing an aliphatic hydrocarbon group and an aromatic hydrocarbon group combined with each other include aralkyl groups such as aralkyl groups having about seven to about eighteen carbon atoms; and alkyl-substituted aryl groups such as phenyl group or naphthyl group having about one to about four alkyl groups each containing about one to about four carbon atoms.

Preferred hydrocarbon groups include alkyl groups having about one to about ten carbon atoms, alkenyl groups having about two to about ten carbon atoms, alkynyl groups having about two to about ten carbon atoms, cycloalkyl groups having about three to about fifteen carbon atoms, aromatic hydrocarbon groups having about six to about ten carbon atoms, cycloalkyl-alkyl groups whose cycloalkyl moiety has about three to about fifteen carbon atoms and whose alkyl moiety has about one to about four carbon atoms, and aralkyl groups having about seven to about fourteen carbon atoms.

These hydrocarbon groups may have one or more substituents. Such substituents include halogen atoms, oxo group, hydroxyl group, substituted oxy groups such as alkoxy groups, aryloxy groups, aralkyloxy groups, and acyloxy groups, carboxyl group, substituted oxycarbonyl groups such as alkoxycarbonyl groups, aryloxycarbonyl groups, and aralkyloxycarbonyl groups, substituted or unsubstituted carbamoyl groups, cyano group, nitro group, substituted or unsubstituted amino groups, sulfo group, and heterocyclic groups. The hydroxyl group and carboxyl group may be protected by protecting groups used in organic syntheses in related art. Rings constituting alicyclic hydrocarbon groups and aromatic hydrocarbon groups may have fused aromatic or nonaromatic heterocyclic rings.

Heterocyclic rings constituting heterocyclic groups as R and other substituents include aromatic heterocyclic rings and nonaromatic heterocyclic rings. Such heterocyclic rings include, for example, heterocyclic rings each containing oxygen atom, sulfur atom, and/or nitrogen atom as a hetero atom. Heterocyclic rings each containing oxygen atom as a hetero atom include five-membered rings such as furan, tetrahydrofuran, oxazole, isoxazole, and gamma-butyrolactone rings; six-membered rings such as 4-oxo-4H-pyran, tetrahydropyran, and morpholine rings; fused rings such as benzofuran, isobenzofuran, 4-oxo-4H-chromene, chroman, and isochroman rings; and bridged rings such as 3-oxatricyclo[4.3.1.1^{4,8}]undecan-2-one ring and 3-oxatricyclo[4.2.1.0^{4,8}]nonan-2-one ring. Heterocyclic rings each containing sulfur atom as a hetero atom include five-membered rings such as thiazole, isothiazole, and thiadiazole rings; six-membered rings such as 4-oxo-4H-thiopyran ring; and fused rings such as benzothiophene ring. Heterocyclic rings containing nitrogen atom as a hetero atom include five-membered rings such as pyrrole, pyrrolidine, pyrazole, imidazole, and triazole rings; six-membered rings such as pyridine, pyridazine, pyrimidine, pyrazine, piperidine, and piperazine rings; and fused rings such as indole, indoline, quinoline, acridine, naphthyridine, quinazoline, and purine rings. These heterocyclic groups may each have one or more substituents. The substituents include, for example, the above-mentioned substituents which the hydrocarbon groups may have, as well as alkyl groups including alkyl groups having about one to about four carbon atoms, such as methyl and ethyl groups; cycloalkyl groups; aryl groups such as phenyl and naphthyl groups.

Preferred as R, R¹, R², R³, and R⁴ are hydrogen atom and hydrocarbon groups such as alkyl groups having about one to about ten carbon atoms, alkenyl groups having about two to about ten carbon atoms, alkynyl groups having about two to about ten carbon atoms, cycloalkyl groups having about three to about fifteen carbon atoms, aromatic hydrocarbon groups having about six to about ten carbon atoms, cycloalkyl-alkyl groups whose cycloalkyl moiety has about three to about twelve carbon atoms and whose alkyl moiety has about one to about four carbon atoms, and aralkyl groups having about seven to about fourteen carbon atoms. Typically preferred as R are phenyl group and alkyl groups having about one to about three carbon atoms, such as methyl group. Typically preferred as R¹, R², R³, and R⁴ are hydrogen atom and alkyl groups having about one to about three carbon atoms, such as methyl group.

Representative examples of allyl ether compounds represented by Formula (1) include allyl ethyl ether, allyl t-butyl ether, 2-butenyl methyl ether, allyloxymethylcyclohexane, 2-butenyl cyclohexyl ether, and 3,6-bis-allyloxy-hexahydro-furo[2,3-b]furan.

An allyl ether compound may be formed within a reaction system and subjected to a reaction according to an embodiment of the present invention. For example, it is acceptable that an allyl ether to be used as a reactant is formed within a reaction system by adding a corresponding allyl ester (R³R⁴C=C(R²)-C(R¹)-OCOR⁵) and a corresponding alcohol (R-OH) to the reaction system. The substituent R⁵ represents an organic group which can be as with the organic groups as R, R¹, R², R³, and R⁴. Representative examples of allyl esters for use herein include allyl acetate, 2-butenyl acetate, 1-methyl-2-butenyl acetate, 2-methyl-2-butenyl acetate, 1,2-dimethyl-2-butenyl acetate, beta-methallyl acetate, allyl formate, allyl propionate, and allyl benzoate. Representative examples of the alcohol include methanol, 2-propanol, allyl alcohol, 2-buten-1-ol, cyclohexyl alcohol, cyclohexylmethyl alcohol, t-butyl alcohol, isosorbide, and phenol.

### [Reaction]

An isomerization reaction of an allyl ether compound represented by Formula (1) may be carried out in the presence of, or in the absence of, a solvent. Examples of the solvent include aliphatic hydrocarbons such as hexane, heptane, and octane; alicyclic hydrocarbons such as cyclohexane; aromatic hydrocarbons such as benzene, toluene, xylenes, and ethylbenzene; halogenated hydrocarbons such as chloroform, dichloromethane, and 1,2-dichloroethane; ethers such as diethyl ether, dimethoxyethane, tetrahydrofuran, and dioxane; ketones such as acetone and methyl ethyl ketone; esters such as methyl acetate, ethyl acetate, isopropyl acetate, and butyl acetate; amides such as N,N-dimethylformamide and N,N-dimethylacetamide; and nitriles such as acetonitrile, propionitrile, and benzonitrile. Each of these solvents can be used alone or in combination.

When an allyl ester compound is used in combination with an alcohol to form an allyl ether compound as a raw material within a reaction system, the amount of the allyl ester compound is, for example, about 0.8 to about 15 equivalents, preferably about 1 to about 12 equivalents, and more preferably about 3 to about 10 equivalents, to 1 equivalent of the alcohol. A reaction may proceed even if the alcohol is used in a catalytic amount. A reaction between an allyl ester compound and an alcohol is preferably carried out in the absence of a base, although it is accelerated in the presence of a transition metal compound such as a Group VIII element compound.

An isomerization reaction may be carried out in the presence of a polymerization inhibitor. The reaction temperature can be set appropriately according typically to the types of reaction components and catalysts and is, for example, about 20°C to about 200°C, preferably about 50°C to about 150°C, and more preferably about 60°C to about 130°C. The reaction may be carried out under ordinary pressure, under reduced pressure, or under a pressure (under a load). The atmosphere of the reaction is not specifically limited, as long as the reaction is not adversely affected, and can be any atmosphere such as air atmosphere, nitrogen atmosphere, or argon atmosphere. The reaction may be conducted according to any system such as a batch system, a semi-batch system, or a continuous system.

By carrying out a process according to an embodiment of the present invention, a corresponding vinyl alcohol represented by Formula (2) is formed under mild conditions. After the completion of the reaction, a reaction product can be separated and purified by a separation procedure such as filtration, concentration, distillation, extraction, crystallization, recrystallization, or column chromatography, or any combination of these separation procedures.

### Examples

The present invention will be illustrated in further detail with reference to several examples below, which by no means limit the scope of the present invention.

### Example 1

There were mixed 10 mmol of allyloxymethylcyclohexane represented by following Formula (1-1), 5 ml of toluene, 0.5 percent by mole of bis(1,5-cyclooctadiene)iridium tetrafluoroborate [Ir(cod)₂]BF₄, and 1 percent by mole of sodium carbonate, and the mixture was stirred at 100°C for five hours. The reaction mixture was analyzed by gas chromatography to find that propenyloxymethylcyclohexane represented by following Formula (2-1) was formed in a yield of 99%.

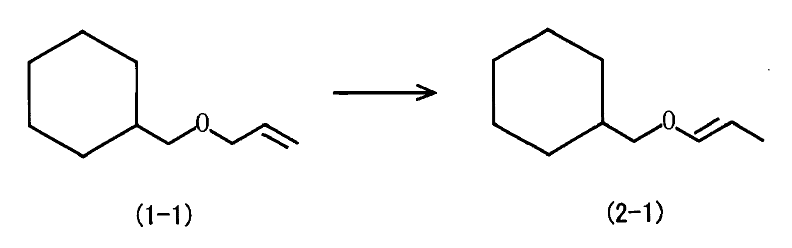

### Example 2

A reaction was carried out by the procedure of Example 1, except for using 1 percent by mole of cesium carbonate instead of sodium carbonate. The reaction mixture was analyzed by gas chromatography to find that propenyloxymethylcyclohexane was formed in a yield of 99%.

### Comparative Example 1

A reaction was carried out by the procedure of Example 1, except for using no sodium carbonate. The reaction mixture was analyzed by gas chromatography to find that propenyloxymethylcyclohexane was formed in a yield of 20%.

### Example 3

There were mixed 10 mmol of allyloxymethylcyclohexane, 5 ml of toluene, 0.5 percent by mole of [Ir(cod)Cl]₂, 0.5 percent by mole of AgBF₄, and 1 percent by mole of sodium carbonate, and the mixture was stirred at 100°C for five hours. The reaction mixture was analyzed by gas chromatography to find that propenyloxymethylcyclohexane was formed in a yield of 90%.

### Example 4

A reaction was carried out by the procedure of Example 3, except for using 1 percent by mole of cesium carbonate instead of sodium carbonate. The reaction mixture was analyzed by gas chromatography to find that propenyloxymethylcyclohexane was formed in a yield of 93%.

### Example 5

A reaction was carried out by the procedure of Example 4, except for using no AgBF₄. The reaction mixture was analyzed by gas chromatography to find that propenyloxymethylcyclohexane was formed in a yield of 93%.

### Example 6

There were mixed 10 mmol of 3,6-bis-allyloxy-hexahydrofuro[3,2-b]furan, 5 ml of toluene, 0.5 percent by mole of bis(1,5-cyclooctadiene)iridium tetrafluoroborate [Ir(cod)₂]BF₄, and 1 percent by mole of sodium carbonate. The mixture was stirred at 100°C for eight hours. The reaction mixture was analyzed by gas chromatography to find that 3,6-bis-propenyloxy-hexahydro-furo[3,2-b]furan was formed in a yield of 95%.

### Example 7

There were mixed 10 mmol of cyclohexylmethyl alcohol, 5 ml of toluene, 40 mmol of allyl acetate, and 1 percent by mole of bis(1,5-cyclooctadiene)iridium tetrafluoroborate [Ir(cod)₂]BF₄. The mixture was stirred at 100°C for ten hours, was combined with 1 percent by mole of sodium carbonate, and was stirred at 100°C for further five hours. The reaction mixture was analyzed by gas chromatography to find that propenyloxycyclohexane was formed in a yield of 52%.

### Example 8

There were mixed 10 mmol of isosorbide, 5 ml of toluene, 60 mmol of allyl acetate, 1 percent by mole of bis(1,5-cyclooctadiene)iridium tetrafluoroborate [Ir(cod)₂]BF₄. The mixture was stirred at 100°C for twelve hours, was combined with 1 percent by mole of sodium carbonate, and was stirred at 100°C for further ten hours. The reaction mixture was analyzed by gas chromatography to find that 3,6-bis-propenyloxy-hexahydro-furo[3,2-b]furan was formed in a yield of 44%.

It should be understood by those skilled in the art that various modifications, combinations, subcombinations, and alterations may occur depending on design requirements and other factors insofar as they are within the scope of the appended claims or the equivalents thereof.

## Claims

1. A process for producing a vinyl ether compound represented by following Formula (2): 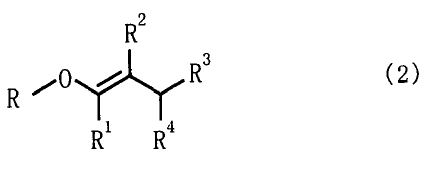 wherein R represents an organic group; and R¹, R², R³ and R⁴ are the same as or different from one another and each represent a hydrogen atom or an organic group,
the process comprising the step of:
isomerizing an allyl ether compound in the presence of a base and a compound of a Group VIII element of the Periodic Table of Elements, the allyl ether compound represented by following Formula (1): 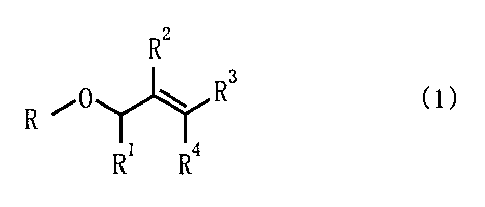
wherein R, R¹, R², R³ and R⁴ are as defined above.

2. The process according to claim 1, wherein the compound of a Group VIII element of the Periodic Table of Elements comprises an iridium compound.
